# EUROPEAN PATENT SPECIFICATION

(11) **EP 1 603 532 B1**
(45) Date of publication and mention of the grant of the patent: **13.08.2008**
(21) Application number: 04720662.8
(22) Date of filing: 15.03.2004
(51) Int. Cl.: A61K 9/00, A61K 9/20, A61K 31/465, A61P 21/00, A61P 25/28, A61P 39/00

(54) **SUBCUTANEOUS DELIVERY SYSTEM, PROCESS FOR THE PREPARATION OF THE SAME AND USE OF THE SAME FOR THE TREATMENT OF CHOLINERGIC DEFICIENT DISORDERS**
SUBKUTANES VERABREICHUNGSSYSTEM, VERFAHREN ZUR HERSTELLUNG DESSELBEN UND VERWENDUNG ZUR BEHANDLUNG VON MANGELERSCHEINUNG DES CHOLINERGEN NERVENSYSTEMS
SYSTEME D'ADMINISTRATION PAR VOIE SOUS-CUTANEE, SON PROCEDE DE PREPARATION, ET SON UTILISATION DANS LE TRAITEMENT DES TROUBLES LIES A UNE DEFICIENCE CHOLINERGIQUE

(30) Priority: 14.03.2003 WO PCT/IB03/00942; 05.11.2003 WO PCT/IB03/04945
(43) Date of publication of application: 14.12.2005
(60) Divisional application: 08159962.3
(73) Proprietor: DEBIO RECHERCHE PHARMACEUTIQUE S.A., 1920 Martigny (CH)
(72) Inventor: MAUVERNAY, Rolland-Yves, CH-1920 Martigny (CH); PORCHET, Hervé, CH-1053 Cugy (CH); SCALFARO, Pietro, CH-1005 Lausanne (CH); HEIMGARTNER, Frédéric, CH-1844 Villeneuve (CH); DUCREY, Bertrand, CH-1920 Martigny (CH); PFEFFERLE, François, CH-1971 Grimisuat (CH); CAPANCIONI, Sergio, CH-1880 Bex (CH); MC CORMICK, Mark, CH-1920 Martigny (CH)
(74) Representative: Grosfillier, Philippe
(86) International application number: PCT/IB2004/000734
(87) International publication number: WO 2004/080436

(56) References cited:
- EP-A- 0 806 416
- DATABASE CHEMABS [Online] CHEMICAL ABSTRACTS SERVICE, COLUMBUS, OHIO, US; LIU, WANHUI ET AL: "Injectable release-sustained microsphere of huperzine A, its derivative, or its salt and its preparation" XP002289907 retrieved from STN Database accession no. 140:327054 & CN 1 393 220 CN (GREEN LEAF PHARMACEUTICAL CO., LTD., SHANDONG, PEOP. REP. CHINA) 3 July 2001 (2001-07-03)
- DATABASE CHEMABS [Online] CHEMICAL ABSTRACTS SERVICE, COLUMBUS, OHIO, US; LIU, WANHUI ET AL: "Injectable sustained-release microspheres of huperzine A compounds" XP002289908 retrieved from STN Database accession no. 138:112410 & WO 03/004024 A1 (SHANDONG LUYE PHARMACEUTICAL CO., LTD., PEOP. REP. CHINA) 16 January 2003 (2003-01-16)
- LALLEMENT G ET AL: "[New prospect for pretreatment of organophosphate intoxication: Huperzine. A. Experimental data.]" MEDECINE ET ARMEES, vol. 30, no. 3, 2002, pages 219-224, XP008033273 ISSN: 0300-4937

## Description

The present invention relates to a subcutaneous delivery system comprising an acetylcholinesterase inhibitor. It relates also to a process for the preparation of said subcutaneous delivery system and to the use of the same for the treatment of neuronal dysfunction, more specifically for the treatment of cholinergic deficient disorders and/or for the improvement of cholinergic dependant functions.

Neuronal dysfunction includes cognitive decline, which is characterised by concentration loss, memory-acquisition loss, and information-storage or retrieval loss. Neuronal dysfunction can also result from central nervous system injury, such as stroke, spinal-cord injury, and peripheral-nerve injury. Cognitive decline is symptomatic of neuronal disorders, such as cognitive decline associated with aging and minimal cognitive impairment, as well as severe neurodegenerative disorders, such as Alzheimer's disease. Neuronal dysfunction is also associated with disorders that result in loss of motor skills, such as Parkinson's disease and amyotrophic lateral sclerosis. It is thought that degeneration of the central cholinergic system contributes to cognitive decline. Alzheimer's disease is the most common form of dementia affecting elderly people with a mean duration of around 8.5 years between onset of clinical symptoms and death. In Alzheimer's disease, neocortical deficits in choline acetyltransferase, the enzyme responsible for the synthesis of acetylcholine (ACh), as well as a reduction of choline uptake and ACh release and loss of cholinergic neurones have been evidenced.

Currently, no curative treatment of Alzheimer's disease exists and symptomatic treatment is focused on augmenting the cholinergic neurotransmission. Several acetylcholinesterase inhibitors are already on the market for the treatment of Alzheimer's disease. These include reversible inhibitors such as tacrine, donepezil, galantamine, and pseudoreversible inhibitors, such as rivastigmine.

All these active agent need to be administered daily by oral route. Such a route for administrating the drug is not appropriate for a regular treatment of patients suffering of memory loss.

Huperzine A (CAS RN: 102518-79-6), a Lycopodium alkaloid isolated from the Chinese herb Huperzia Serrata, is a promising agent since the first report of its anticholinesterase activity by Wang et al. in Zhongguo Yaoli Xuebao, 1986, 7, 110-3. It is now recognised as being a reversible, highly selective acetylcholinesterase inhibitor and has shown memory-enhancing effects in aged patients and patients suffering from Alzheimer's disease. Furthermore, in vitro and in vivo data document that Huperzine A has in addition neuroprotective properties. Since the report of the pharmacological properties of Huperzine A, a number of Huperzine A derivatives have been synthesised in order to identify more potent products. Zhu et al. described in patent application EP 0 806 416 very promising Huperzine A derivatives, in particular those being considered as Schiff bases. Such compounds demonstrate to have better treatment effect and lower toxicity than Huperzine A. In particular, Huperzine A derivative of formula (III) 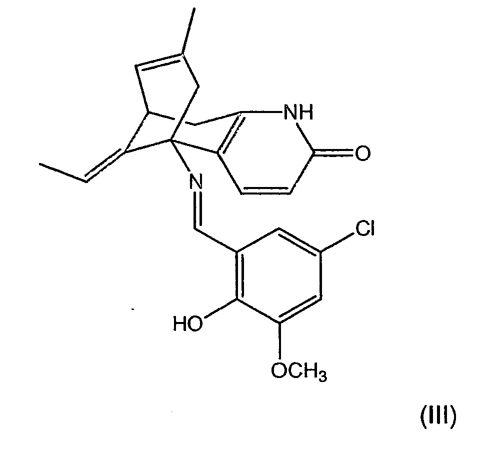 and having CAS Registry number 180694-97-7, demonstrates to have a highly selective activity in favour of acetylcholinesterase compared to butyrylcholinesterase. One of the drawbacks of those Huperzine A derivatives is their sensitivity to water precluding any manipulation using uncontrolled conditions in contact of water.

One of the proposed administration route for Huperzine A treatment is the oral route. However, one of the inconvenient aspects of such a treatment is its frequent administration, two to four times a day or even more, which can hardly be followed scrupulously to the letter by a patient suffering from Alzheimer's disease. One of the other inconvenient aspects of such a treatment is the variability in exposure leading to less marked neuroprotection when studying diverse types of cerebral insults. Such variabilities are, in particular, dependent or influenced by food absorption and by fluctuations in Huperzine A plasma levels.

Besides oral administration, different further routes have been investigated.

For instance, in patent application CN 1383824, Wang et al. propose, as a treatment of senile dementia and memory disorder or for improving memory functions, to administrate Huperzine A via the nasal cavity by using, for instance, nasal sprays, nasal drops, ointments, gels, powders, or microspheres. They mention that each administration may deliver between 80 to 500 micrograms of Huperzine A into the body. A similar nasal drug delivery system is proposed by Zhang in patent application CN 1279065. Such a route for administrating a drug allows an immediate penetration through mucosa into blood and a rapid brain targeting. However, the dose to be administrated can hardly be monitored and the administration needs to be repeated along the day. For the very similar reasons as above, such a route is not appropriate for a regular treatment of patients suffering of concentration and memory losses.

In patent US 6,352,715, Kou et al. propose to patients with Alzheimer disease to apply a transdermal delivery device designed in such a way that it provides a controlled release of Huperzine A over a period of 7 days at a therapeutically effective rate of no less than 0.833 to 146 µg/cm².h. Such controlled release skin patches designed for a once-a-week application are recognised to offer therapeutical benefits to patients in full possession of all their faculties. However, due to their locally irritating effects and the fact that patients suffering of memory loss and behavioural problems may rip them off and thus interfere negatively with the treatment.

In International patent application WO 03/04024, Liu et al. propose to treat a patient suffering from Alzheimer disease by injecting every two weeks Huperzine A encapsulated into sustained-release microspheres, comprising, as the biodegradable polymer, poly(lactide-co-glycolide). Those microspheres are obtained according two classical methods, the first one being known as "emulsion-evaporation" process using, as the solvents, dichloromethane and water, and the second one being known as "spray drying" process. Considering the drug release profile as obtained with these microspheres, it appears that Huperzine A is released over a period of at least 17 days, but following two separate phases. Almost 40% of the amount of Huperzine A encapsulated in the microspheres are released during Day 1 and Day 2, the remaining amount of the drug being released mainly between Day 8 and Day 15. Such a two phases release profile, with an important burst in drug released immediately after injection, may provoke important deleterious side effects due to an over exposition to the acetylcholinesterase inhibitor during a very short period of time and considering the narrow therapeutic index of such a drug.

From the review of the state of the art, it is appears that there is still a need to provide the clinician with a galenic formulation allowing a gradually and controlled release of Huperzine A and/or some of its pharmacologically active substances analogs over a period varying from several weeks to several months under a minimal control of a health professional and without requesting the patient any contribution and avoiding self-medication.

Surprisingly, those goals have been successfully reached with the delivery system of the present invention.

Accordingly, the object of the present invention relates to subcutaneous delivery system under the form of a solid in plant which is an extrudate comprising a biodegradable polymeric matrix and at least one of the pharmacologically active substances of general formula (I): 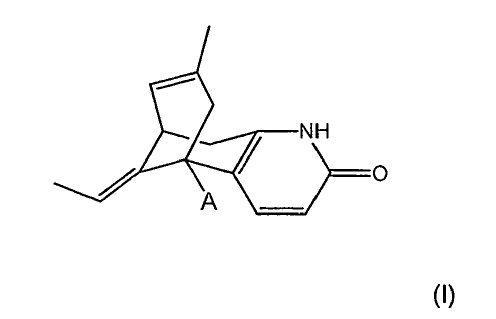 wherein residue A represents an amino group -NH₂ or an ammonium group -NH₃⁺ or a residue of general formula (II): 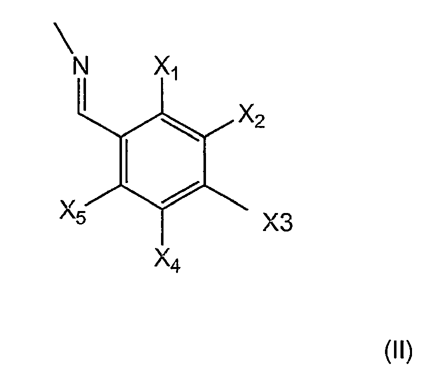 wherein each of X₁ to X₅ represents, independently, an hydrogen atom, a linear or branched C₁ to C₆ alkyl group, a linear or branched C₁ to C₆ alkyloxy group, a hydroxyl group -OH, an amino group -NH₂, a primary or secondary C₁ to C₆ alkylamino group, a halogen atom, a nitro group -NO₂; said substance being embedded into said matrix.

As the pharmacologically active substances defined by general formula (I) possess asymmetry, the present definition encompasses any of the possible optically pure isomers, and any mixtures of said optically pure isomers. Preferably, the asymmetric carbon atom carrying residue A has essentially the absolute configuration R (with reference with A being a group amino -NH₂).

When, in general formula (I), residue A represents an ammonium group -NH₃⁺, the corresponding substance is present in the delivery system along with an appropriate pharmacologically acceptable counter-ion, such as, for instance, a halide, preferably a chlorine anion.

When, in general formula (I), residue A represents an amino group -NH₂, the so-defined pharmacologically active substance is Huperzine A, preferably (-)-Huperzine A. (-)-Huperzine A may be obtained indifferently either by extraction from plants, for instance Huperzia Serrata, or by total synthesis or hemisynthesis. Both types of methods are already known from the literature.

When, in general formula (I), residue A represents a residue of general formula (II), the so-defined pharmacologically active substances are obtainable by applying methods as described in patent application EP 0 806 416 or by analogy with such methods, by condensation between Huperzine A and the appropriate moieties having an aldehyde function, leading to the corresponding Schiff base.

Preferably, residue A represents an amino group -NH₂ or an ammonium group -NH₃⁺ or a residue of general formula (II): 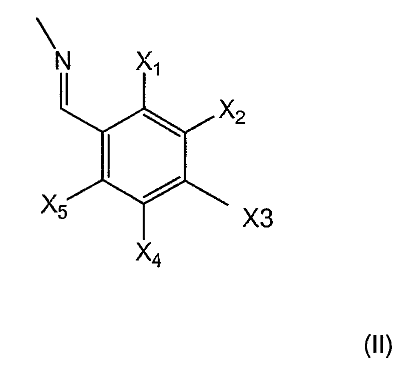 wherein each of X₁ to X₅ represents, independently, an hydrogen atom, a methyl or ethyl group, a methoxy group, a hydroxyl group -OH, an amino group -NH₂, a methylamino group or a dimethylamino group, a chlorine or a bromine atom, a nitro group -NO₂.

More preferably, the pharmacologically active substance has the following formula (III):

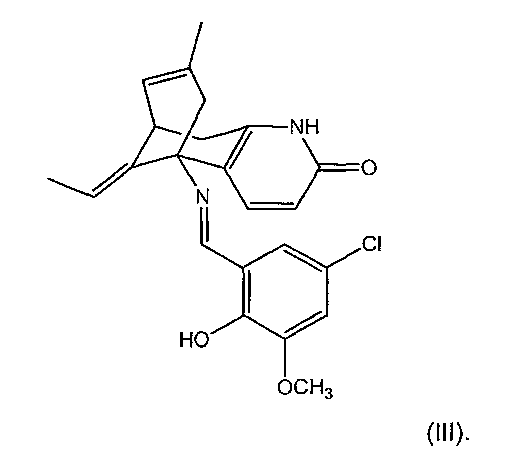

The main pharmacological effect of the above-defined pharmacologically active substance, especially the one of formula (III), is their capability for inhibiting cholinesterases. Moreover, substance of formula (III) appears to be a potent and selective inhibitor of acetylcholinesterase (AChE) in different species (rat, bovine and human). In contrast, it presents a much weaker inhibitory activity on butyrylcholinesterase (BuChE). These enzymes are responsible for the breakdown of the neurotransmitter acetylcholine (ACh). The inhibition of cholinesterases leads therefore to an increase in ACh with an increase in cholinergic activity in the brain and to a lesser extent also in peripheral target organs (e.g. muscles, conducting autonomous nervous system, gastrointestinal system).

This inhibitory profile confers specially to substance of formula (III) the potential to be beneficial in cholinergic deficient disorders (e.g. Alzheimer's disease, Mysthenia gravis, Dementia of other origins such as vascular), or to improve cognition in cholinergic dependant functions (e.g. attention, memory, concentration, perception, learning) and therefore the potential to be beneficial in premorbid states (such as minimal cognitive impairment) or in conditions where a sustained improvement in cognition is beneficial (e.g. pilots, etc.). In addition, the reversible preventive selective inhibition of cholinesterase with a preference for central inhibition might protect persons intoxicated, for instance by organophosphates.

In vitro and in vivo data document that Huperzine A has in addition neuroprotective properties. This dual pharmacological mechanism of action confers to the pharmacologically active substances of general formula (I) a potential for neuroprotection in a variety of cerebral disorders. These effects might be mediated through the modulation of the N-methyl-D-aspartate (NMDA) receptor and other mechanisms. NMDA receptors are widely distributed in the brain and play an important role in brain development, memory formation and learning. Moreover, NMDA receptor activity could contribute to the aetiology of many neurodegenerative diseases, through mechanisms described as excitotoxicity. Excitotoxicity consists in an uncontrolled synaptic overactivity leading to the enhanced release of excitatory neurotransmitters. Glutamate is a dominating excitatory neurotransmitter in cortical and hippocampal neurones. It triggers a signalling cascade by activating the postsynaptic NMDA receptors. Once activated the opening of their associated ion channel pore leads to a Ca2+ influx into the cell. An excess of intracellular Ca2+ ions then activates Ca2+-dependant pathways that can lead to neuronal cell death.

Many studies showed that different neuropathological conditions could be improved by blocking the NMDA receptors. In the Alzheimer brain increased glutamate levels cause partial depolarisation of the NMDA receptors resulting in increased Ca2+ influx. The sustained and constant prevention of this Ca2+ influx can lead to a better clinical outcome in AD and other neurodegenerative diseases where excitotoxicity is involved. In addition a beneficial neuroprotective effect of Huperzine A rescue treatment was shown in a cerebral hypoxia-ischemia rat model. The decreased neuronal cell death was significantly related to a reduced cognitive impairment.

In the delivery system of the present invention, the matrix is made of a biodegradable polymeric material and this material is selected among the group comprising: polyacetals and poly(hydroxycarboxylic esters), polyorthoesters, polyanhydrides, polylactones, or a mixture thereof. Preferably, the poly(hydroxycarboxylic esters) are selected among the group comprising homopolymers and copolymers of D-lactic acid and/or L-lactic acid and/or glycolic acid; and block-polymers thereof with polyethylene glycol; and the polylactones are selected among the group comprising polycaprolactone, poly(3-hydroxy-butyrolactone), and hydroxybutyrolactone-hydroxyvalerolactone copolymer.

More preferably, the biodegradable polymeric material is selected among the group comprising polylactic acid, and copolymers of D-lactic acid and/or L-lactic acid, and/or D,L-lactic acid, and/or glycolic acid. More preferably, the biodegradable polymeric material is selected among the group consisting of poly(D,L-lactide-co-glycolide) and polylactide. The proportion between D,L-lactide and glycolide constituting the biodegradable polymeric material is comprised in the range of 25:75 to 100:0 mol%, preferably 50:50 to 75:25 mol%. The inherent viscosity of the biodegradable polymeric material is comprised in the range of 0.10 to 0.9 dL/g, preferably 0.15 to 0.6 dL/g. Ratios are given following NMR spectra recordation of the polymer in question and the given values must be considered within the usual margins of error. Inherent viscosities are measured at a temperature of 25°C, the polymer being in solution in chloroform at a concentration of 0.5 g/dL and the given values must be considered within the usual margins of error. By an appropriate compromise within the above proposed ranges between, on the one hand, the proportion of D,L-lactide and glycolide in the poly(D,L-lactide-co-glycolide) polymer, and, on the other hand, the inherent viscosity, the specialist of the art can easily select the appropriate poly(D,L-lactide-co-glycolide) polymer to be used as the matrix of the delivery system of the present invention and allowing a gradually and controlled release of the pharmacologically active substances of general formula (I) over a period of at least one month. Generally, the selected polymer is commercially available. For instance, Boehringer Ingelheim, Germany, provides with a poly(D,L-lactide-co-glycolide) polymer under the trade name Resomer® having a molar ratio D,L-lactide/glycolide of 75:25 and an inherent viscosity comprised between 0.14 and 0.22 dL/g and Mitsui Chemicals, Japan, provides with a poly(D,L-lactide-co-glycolide) polymer under the trade name PLGA 5-50 having a molar ratio D,L-lactide/glycolide of 50:50 and an inherent viscosity comprised between 0.47 and 0.53 dL/g.

The delivery system of the present invention has the form of a solid implant. Any shape for this solid implant may be contemplated. Preferably, this solid implant has a cylindrical shape.

One of the other objects of the present invention is a process for the preparation of the present delivery system. That process comprising the following steps:
a) preparing a homogenous admixture an appropriate amount of dry powder of said at least one of the pharmacologically active substances of general formula (I) and an appropriate amount of dry powder of said biodegradable polymeric material;
b) extruding the admixture as obtained in step a) at a controlled rate through a die with an appropriate circular section placed at temperatures ranging from 60°C to 120°C;
c) cutting the extrudate as obtained in step b) essentially through the section of said extrudate and at an appropriate length in order to obtain the solid implants; and
d) sterilising the implants as obtained in step c).

By reference to the pharmacologically active substance of formula (III) and when the polymeric material is a poly(D,L-lactide-co-glycolide) polymer, the relative proportion of that active substance, in weight % with reference to the total weight of the obtained implant, is comprised between 20 to 60%, preferably 25 to 35%. The appropriate size of the respective powders may be easily selected by the specialist. Preferably, during step b) of the present process, the temperature is comprised between 60 and 100°C, more preferably between 60°C and 90°C. Preferably, the internal diameter of the die is less than 1.6 mm, and the obtained extrudate is cut through its section at a length comprised between 2 and 6 mm.

This process is particularly convenient for the preparation of a delivery system according to the present invention comprising a pharmacologically active substance of general formula (I) in which A represent a residue of formula (II) as it avoids at any of the steps the use or the presence of water, so precluding any, or almost any degradation of said substance either in the course of the process or once the delivery system has been obtained. The obtained delivery system appears to fulfil the pharmacopoeia stability criteria and can be stored for months at room temperature (around 25°C).

One of the other objects of the present invention is the use of the delivery system as defined above for the preparation of a medicament for the treatment of a person suffering from cholinergic deficient disorders and/or for the improvement of cholinergic dependent functions in a person. Those disorders comprise Alzheimer disease, mild cognitive impairment, myasthenia gravis, dementia, dementia of vascular origin; and those cognition functions are involved in human beings processes selected among the group comprising perception, attention, learning, memory, thought, concept formation, reading, problem solving, and language.

In the use of the present invention, the medicament is also intended for the prevention and/or for the treatment of a person subject to, and/or intoxicated by agents with a cholinesterase inhibitory activity. Most of those agents are selected from the group of organophosphates.

The delivery system of the present invention is injected just under the skin in the abdominal area or other typically used sites for subcutaneous administration of medicinal products (e.g. arm, thigh). A health professional will fold the skin on the abdomen and insert the needle underneath the skin and inject the implant. Prior to injection a local anesthetic may be applied to reduce possible discomfort.

The delivery system of the present invention is conceived to be injected at regular intervals from weeks to months, preferably at intervals of at least one month and possibly 3 months or even more, and cover a dose range of 3 mg to 50 mg with reference to substance of formula (III). Dose can be adjusted to patient response and need with an appropriate injection device, for instance any trocar devices commercially available. This device will allow a simple, adjustable injection of an individualized dose by providing several implants through one injection site.

One of the further advantage of a subcutaneous injection resides in the fact that it is generally better tolerated than the intramuscular injection route used for instance with the microspheres similar to those of the prior art. Furthermore, the effects of the pharmacologically active substance may be reversed if therapy needs to be discontinued. Localization of the implant through the skin can be done as compared to microspheres that are injected intramuscularly. The subcutaneous route allows also an easy surveillance of local tolerance. The implant can be extracted by a simple and minimally invasive surgical procedure in case of a local or general intolerance.

The delivery system of the present invention, their preparations and their properties are described in the following Examples.

In the Examples,
- Fig 1 represents pharmacokinetic profile of substance of formula (III) in rats following a single s.c. administration of sustained release formulation of Example 1; and
- Fig 2 represents pharmacokinetic profile of derivative of formula (III) in rats following a single s.c. administration of sustained release formulation of
Example 2.

### Example 1:

The following procedure, known as dry-extrusion, was used to produce implants composed of poly(D,L-lactide-co-glycolide) polymer and derivative of formula (III).

First, a mixture was prepared by mixing 14.0 g of ground (500 µm) poly(D,L-lactide-co-glycolide) (molar ratio D,L-lactide/glycolide: 54/51 to 46/49 mol%; inherent viscosity: 0.47-0.53 dL/g in chloroform (0.5g/dl in CHCl₃) at 25°C; known under the denomination of PLGA 5-50, Mitsui Chemicals Inc.) and 6.0 g of derivative of formula (III) in a ball mill (400 rpm, 4 min.). Next, the obtained mixture was extruded through successive zones at temperatures of 70, 80, 90, 90°C through a 1.3 mm diameter die using a screw-type extruder. The resultant extrudate, containing derivative of formula (III) (theoretical core loading 30% w/w), was cut into implants (average diameter of 1.5 mm) and sterilized by gamma irradiation (25 kGy).

The implants were tested in rats according to the following method. A group of six 10-11 week-old Sprague-Dawley male rats were given a single subcutaneous administration of an implant (dose: ca 15 mg/kg of derivative of formula (III), i.e. 37 µmol/kg) under the neck skin by using a trocar.

The day before administration, a reference plasma sample was collected. Plasma samples for drug measurement were then collected at 2h, 6h, 8h, 10h post-dosing and then twice a week from day 2 to day 35 after administration.

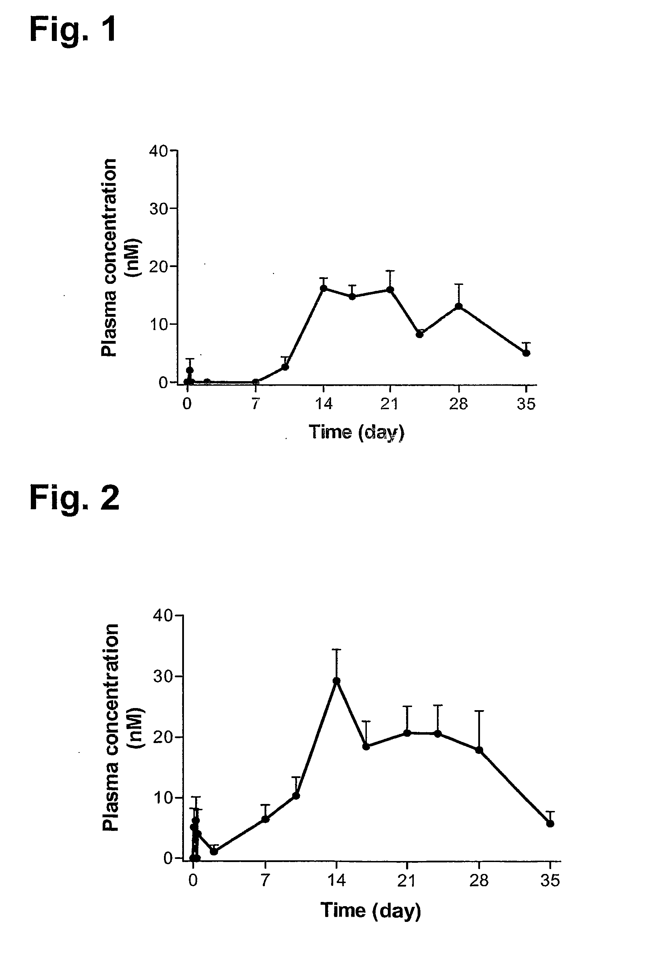

Plasma levels of both derivative of formula (III) and in vivo generated Huperzine A were simultaneously quantified in rat plasma samples by LC/MS/MS method using a reducing agent such NaBH₄ as an inhibitor of the Schiff base hydrolysis. Time concentration-curves were established for each rat and recorded on Fig. 1. Mean pharmacokinetic profiles were calculated (± SEM, n=6). In the meantime, animals were observed for clinical signs throughout the study. At the end of the study, animals were sacrificed and the site of implantation was recovered for evaluation of local inflammation and implant degradation.

The following conclusions can be made. A progressive release of Huperzine A (generated by in vivo hydrolysis of derivative of formula (III)) in plasma was observed between Day 7 and Day 35, Day 0 being the administration day. Sustained Huperzine A plasma levels were achieved over a 1 month-period, while levels of derivative of formula (III) were negligible. Inter-individual variability was low. No clinical signs were observed throughout the study and no sign of local intolerance were reported. Implants were almost completely degraded within 35 days after administration.

### Example 2:

The following procedure, similar as the one described in Example 1, was used to produce implants, but with a different poly(D,L-lactide-co-glycolide) polymer.

First, a mixture was prepared by mixing 14.0 g of ground poly(D,L-lactide-co-glycolide) (molar ratio D,L-lactide/glycolide: 76/74 to 24/26; inherent viscosity: 0.16-0.20 dL/g, (0.1 % in chloroform at 25°C); known under the denomination of Resomer RG 75:25H, Boehringer Ingelheim) and 6.0 g of derivative of formula (III) in a ball mill (400 rpm, 4 min.). Next, the obtained mixture was extruded through successive zones at temperatures of 60, 70, 80, 75°C through a 1.3 mm diameter die using a screw-type extruder. The resultant extrudate; containing derivative of formula (III) (theoretical core loading 30% w/w), was cut into implants (average diameter of 1.3 mm) and sterilized by gamma irradiation (25 kGy).

The implants were tested in rats according to the method as described in Example 1 and respective time concentration-curves were established for each rat and recorded on Fig. 2. Very similar conclusions can be made as for Example 1.

The sustained release formulations prepared according to the following processes appear to fulfil all the aims of the present invention.

### Example 3

Implants composed of poly(D,L-lactide-co-glycolide) polymer and derivative of formula (III) were produced by extrusion of a film according to the following procedure.

First, water was removed from ethyl acetate by suspending anhydrous magnesium sulphate in the solvent and subsequently removing the desiccant by filtration. 600 mg of derivative of formula (III) was dissolved in 10.0 g of ethyl acetate. A polymer suspension was prepared by suspending 1.40 g of poly(D,L-lactide-co-glycolide) 50:50 polymer in 80.0 g of ethyl acetate. The suspension was subjected to ultrasound for 15 minutes. The solution of derivative of formula (III) and the polymer suspension were placed in a round flask. The round flask was heated to 37°C and rotated at 75 rpm thereby removing approximately 90% of the solvent by evaporation. The viscous suspension was poured onto a Teflon sheet, dried under a fume hood and then under vacuum in a drying oven at room temperature to form a film. The weight of the resulting film was 1.64 g.

The film was cut into pieces and extruded at a temperature of 54°C through a 0.8 mm die using a press extruder. The resultant extrudate was cut into implants and sterilized by gamma irradiation. The implants had an average diameter of 1.30 mm and comprised 24.80 wt. % derivative of formula (III). The incorporated active principle was 95.06% pure.

### Example 4

The procedure, known as dry-extrusion, was used to produce implants composed of poly(D,L-lactide-co-glycolide) polymer and derivative of formula (III).

First, poly(D,L-lactide-co-glycolide) 50:50 polymer was ground to obtain microgranules. Next, a physical mixture was prepared by mixing 1400 mg of the ground polymer and 600 mg of derivative of formula (III) using a mortar and pestle. The physical mixture was extruded at a temperature of 71-77°C through a 0.8 mm die using a press-type extruder. The resultant extrudate weighed 1.3 g.

The extrudate was cut into implants and sterilized by gamma irradiation. The implants had an average diameter of 1.3 mm and comprised 30 (w/w) derivative of formula (III). The incorporated active principle was 97.56% pure. The implants were administered subcutaneously in rats.

### Example 5

The procedure, known as dry-extrusion, was used to produce implants composed of poly (D,L-lactide-co-glycolide) polymer and Derivative of formula (III).

First, a physical mixture was prepared by mixing 1400 mg of poly(D,L-lactide-co-glycolide) 50:50 polymer, known as Resomer® RG 503H (D,L-lactide: 48-52 mol%; glycolide. 48-52 mol%; Inherent viscosity: 0.1 % in CHCl₃ at 25°C: 0.32-0.44 dL/g)) and 600 mg of derivative of formula (III) using a mortar and pestle. The physical mixture was extruded at a temperature of 74°C through a 0.8 mm die using a press-type extruder. The resultant extrudate weighed 1.320 g. The extrudate was cut into implants and sterilized by gamma irradiation. The implants had an average diameter of 1.40 mm and comprised 27.65 wt. % derivative of formula (III). The incorporated active principle was 97.37% pure. The implants were administered subcutaneously in rats.

### Example 6

Implants composed of poly(D,L-lactide-co-glycolide) polymer and derivative of formula (III) were produced by extrusion of a film according to the following procedure.

First, water was removed from ethyl acetate by suspending anhydrous magnesium sulphate in the solvent and subsequently removing the desiccant by filtration. 900 mg of derivative of formula (III) was dissolved in 20.0 g of ethyl acetate. A polymer solution was prepared by dissolving 2.10 g of poly (D,L-lactide-co-glycolide) 50:50 lauryl ester end-capped polymer in 20.0 g of ethyl acetate. The two solutions were placed in a round flask that was rotated at 75 rpm. The solution was heated to 37°C. The weight of the solution was reduced by 33 g by evaporation of the solvent. The viscous solution was poured onto a Teflon sheet. The viscous solution was dried under a fume hood and then under vacuum in a drying oven at room temperature to form a film. The weight of the resulting film was 2.77 g.

The film was cut into pieces and extruded at a temperature of 38 - 49°C through a 0.8 mm die using a press extruder. The resultant extrudate weighed 1.18 g. The extrudate was cut into implants and sterilized by gamma irradiation. The implants had an average diameter of 1.2 mm and comprised 26.50 %(w/w) derivative of formula (III). The incorporated active principle was 94.74% pure. The implants were administered subcutaneously in rats.

### Example 7

The procedure, known as dry-extrusion, was used to produce implants composed of poly(lactide-co-glycolide) polymer and derivative of formula (III).

First, poly(D,L-lactide-co-glycolide) 50:50 lauryl ester end-capped polymer was ground to obtain microgranules. A physical mixture was prepared by mixing 1.400 g of ground polymer and 600 mg of derivative of formula (III) using a mortar and pestle. The physical mixture was extruded at a temperature of 65°C through a 0.8 mm die using a press-type extruder. The resultant extrudate weighed 1.170 g.

The extrudate was cut into implants and sterilized by gamma irradiation. The implants had an average diameter of 1.3 mm and comprised 27.59 wt. % derivative of formula (III). The incorporated active principle was 97.43% pure. The implants were administered subcutaneously in rats.

### Example 8

Implants composed of poly(D,L-lactide-co-glycolide) polymer and derivative of formula (III) were produced by extrusion of a film according to the following procedure.

First, water was removed from ethyl acetate by suspending anhydrous magnesium sulphate in the solvent and subsequently removing the desiccant by filtration. Next, 600 mg of derivative of formula (III) was dissolved in 14.8 g of ethyl acetate. Next, a polymer solution was prepared by dissolving 1.4 g of poly(D,L-lactide-co-glycolide) 75:25 polymer (known as Resomer® RG 75:25H; molar ratio D,L-lactide/glycolide: 76/74 to 24/26; inherent viscosity: 0.16-0.20 dL/g, (0.1 % in chloroform at 25°C) in 15.2 g of ethyl acetate. The solution of derivative of formula (III) and the polymer solution were placed in a round flask. The round flask was heated to 37°C and rotated at 75 rpm thereby removing approximately 90% of the solvent by evaporation. The viscous suspension was poured onto a Teflon sheet, dried under a fume hood and then under vacuum in a drying oven at room temperature to form a film. The weight of the resulting film was 1.56 g.

The film was cut into pieces and extruded at a temperature of 59°C through a 0.8 mm die using a press extruder. The resultant extrudate was cut into implants and sterilized by gamma irradiation. The diameter of the implants was approximately 1.3 mm. The implants comprised 24.8 wt. % derivative of formula (III). The incorporated active principle was 92.59% pure. The implants were administered subcutaneously in rats.

### Example 9

The procedure, known as dry-extrusion, was used to produce implants composed of poly(D,L-lactide-co-glycolide) polymer and derivative of formula (III).

First, a physical mixture was prepared by mixing 1400 mg of poly(D,L-lactide-co-glycolide) 75:25 polymer (known as Resomer® RG 75:25H; molar ratio D,L-lactide/glycolide: 76/74 to 24/26; inherent viscosity: 0.16-0.20 dL/g, (0.1 % in chloroform at 25°C) and 600 mg of derivative of formula (III) using a mortar and pestle. The physical mixture was extruded at a temperature of 65°C through a 0.8 mm die using a press-type extruder. The resultant extrudate weighed 1.283 g.

The resultant extrudate was cut into implants and sterilized by gamma irradiation. The implants had an average diameter of 1.5 mm and comprised 27.2 wt. % derivative of formula (III). The incorporated active principle was 97.3% pure.

### Example 10

Implants were produced by extrusion of microspheres.

3.5 g of poly(D,L-lactide-co-glycolide) 50:50 polymer, known as Resomer RG 503 H (D,L-lactide: 48-52 mol%; glycolide. 48-52 mol%; Inherent viscosity: 0.1 % in CHCl₃ at 25°C: 0.32-0.44 dL/g), were dissolved in 60g dichloromethane. 1.5g of derivative of formula (III) were dissolved in 35g of dichloromethane, then poured into the polymer solution. The obtained mixture was sprayed using a mini spray-dryer device at a temperature of 45°C leading to the formation of microspheres.

Implants of different diameters were formed by extrusion of these microspheres through 1.0 to 2.0 mm diameter dies at temperatures in between 60-95°C.

The resultant extrudate was cut into implants and sterilized by gamma irradiation. The implants were administered subcutaneously.

### Example 11

Implants were produced by extrusion of microspheres.

3.0 g of poly(D,L-lactide) ester end-capped polymer (Inherent viscosity of 0.09-0.10 dL/g measured at 0.5 g/dL in CHCl₃ at 30°C obtained from Absorbable Polymer Technologies) were dissolved in 60 g dichloromethane. 2.0 g of derivative of formula (III) were dissolved in 35g of dichloromethane, then poured into the polymer solution. The obtained mixture was sprayed using a mini spray-dryer device at a temperature of 40°C leading to the formation of microspheres.

Implants of different diameters were formed by extrusion of these microspheres through 1.0 to 2.0 mm diameter dies at temperatures in between 60-95°C.

The resultant extrudate was cut into implants and sterilized by gamma irradiation. The implants were administered subcutaneously.

### Example 12

Implants were produced by extrusion of microspheres.

3.0 g of poly(D,L-lactide) polymer (free COOH, Inherent viscosity 0.09-0.10 dL/g measured at 0.5 g/dL in CHCl₃, at 30°C obtained from Absorbable Polymer Technologies) were dissolved in 60 g dichloromethane. 2.0 g of derivative of formula (III) were dissolved in 35g of dichloromethane, then poured into the polymer solution. The obtained mixture was sprayed using mini spray-dryer device at a temperature of 40°C leading to the formation of microspheres.

Implants of different diameters were formed by extrusion of these microspheres through 1.0 to 2.0 mm diameter dies at temperatures in between 60-95°C.

The resultant extrudate was cut into implants and sterilized by gamma irradiation.

### Example 13

The procedure, known as dry-extrusion, was used to produce implants composed of polyD,L-(lactide) polymer and derivative of formula (III).

First, a physical mixture was prepared by mixing 2.1 g of grounded poly(D,L-lactide) ester end-capped polymer (Inherent viscosity 0.09-0.10 dL/g measured at 0.5 g/dL in CHCl₃ at 30°C obtained from Absorbable Polymer Technologies) and 900 mg of derivative of formula (III) using a mortar and pestle. The physical mixture was extruded at a temperature of 30-50°C through 1.0-2.0 mm dies using a press-type extruder.

The resultant extrudate was cut into implants and sterilized by gamma irradiation. The implants were administered subcutaneously.

### Example 14

The procedure, known as dry-extrusion, was used to produce implants composed of poly(D,L-lactide) polymer and derivative of formula (III).

First, a physical mixture was prepared by mixing 2.1 g of grounded poly(D,L-lactide) polymer (free COOH, Inherent viscosity of 0.09-0.10 dL/g measured at 0.5 g/dL in CHCl₃ at 30°C obtained from Absorbable Polymer Technologies) and 900 mg of derivative of formula (III) using a mortar and pestle. The physical mixture was extruded at a temperature of 30-50°C through 1.0-2.0 mm dies using a press-type extruder.

The resultant extrudate was cut into implants and sterilized by gamma irradiation. The implants were administered subcutaneously.

## Claims

1. A subcutaneous delivery system in the form of a solid implant which is an extrudate comprising a biodegradable polymeric matrix and at least one of the pharmacologically active substances of general formula (I): 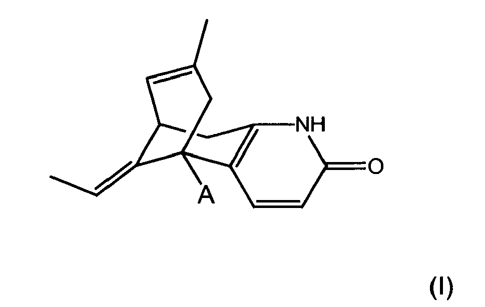 wherein residue A represents an amino group -NH₂ or an ammonium group -NH₃⁺ or a residue of general formula (II): 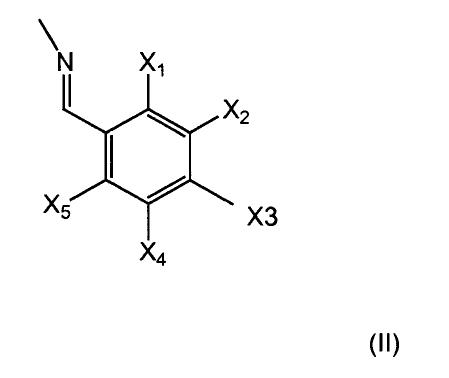 wherein each of X₁ to X₅ represents, independently, an hydrogen atom, a linear or branched C₁ to C₆ alkyl group, a linear or branched C₁ to C₆ alkyloxy group, a hydroxyl group -OH, an amino group -NH₂, a primary or secondary C₁ to C₆ alkylamino group, a halogen atom, a nitro group -NO₂; said substance being embedded into said matrix.

2. The delivery system according to claim 1, **characterised in that**, in general formula (I) defining said pharmacologically active substance, residue A represents an amino group -NH₂ or an ammonium group -NH₃⁺ or a residue of general formula (II): 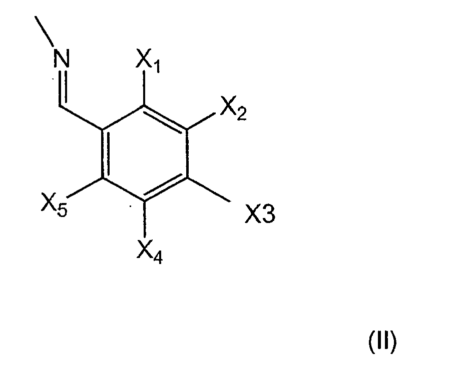 wherein each of X₁ to X₅ represents, independently, an hydrogen atom, a methyl or ethyl group, a methoxy group, a hydroxyl group -OH, an amino group -NH₂, a methylamino group or a dimethylamino group, a chlorine or a bromine atom, a nitro group -NO₂.

3. The delivery system according to claim 1, **characterised in that** said pharmacologically active substance has the following formula (III):

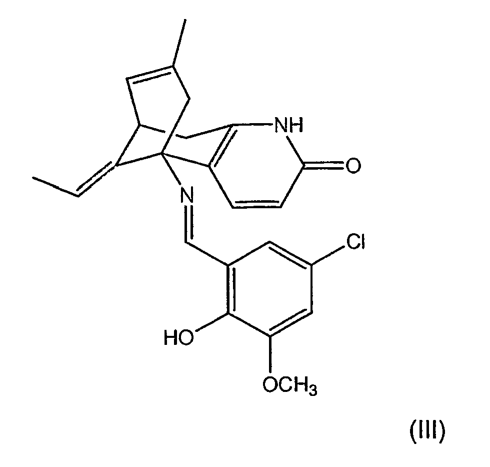

4. The delivery system according to claim 1, **characterised in that** the biodegradable polymeric material of said matrix is selected among the group comprising: polyacetals and poly(hydroxycarboxylic esters), polyorthoesters, polyanhydrides, polylactones, or a mixture thereof.

5. The delivery system according to claim 4, **characterised in that** said poly(hydroxycarboxylic esters) are selected among the group comprising homopolymers and copolymers of D-lactic acid and/or L-lactic acid and/or glycolic acid; and block-polymers thereof with polyethylene glycol; and said polylactones are selected among the group comprising polycaprolactone, poly(3-hydroxybutyrolactone), and hydroxybutyrolactone-hydroxyvalerolactone copolymer.

6. The delivery system according to claim 4, **characterised in that** said biodegradable polymeric material is selected among the group comprising polylactic acid, and copolymers of D-lactic acid and/or L-lactic acid, and/or D,L-lactic acid, and/or glycolic acid.

7. The delivery system according to claim 6, **characterized in that** said biodegradable polymeric material is selected among the group consisting of poly(D,L-lactide-co-glycolide) and polylactide and the proportion between D,L-lactide and glycolide constituting the biodegradable polymeric material is comprised in the range of 25:75 to 100:0 mol%, preferably 50:50 to 75:25 mol%; the inherent viscosity of the biodegradable polymeric material is comprised in the range of 0.10 to 0.9 dL/g, preferably 0.15 to 0.6 dUg.

8. The delivery system according to claim 1, **characterised in that** said solid implant has a cylindrical shape.

9. A process for the preparation of the delivery system as defined in claim 4 comprising the following steps:
a) preparing a homogenous admixture an appropriate amount of dry powder of said at least one of the pharmacologically active substances of general formula (I) and an appropriate amount of dry powder of said biodegradable polymeric material;
b) extruding the admixture as obtained in step a) at a controlled rate through a die with an appropriate circular section placed at temperatures ranging from 70°C to 100°C;
c) cutting the extrudate as obtained in step b) essentially through the section of said extrudate and at an appropriate length; and
d) sterilising the implants as obtained in step c).

10. Use of the delivery system as defined in claim 1 for the preparation of a medicament for the treatment of a person suffering from cholinergic deficient disorders and/or for the improvement of cholinergic dependent functions in a person.

11. The use according to claim 10, **characterised in that** said disorders comprise Alzheimer disease, mild cognitive impairment, myasthenia gravis, dementia, dementia of vascular origin; and said cognition functions are involved in human being processes selected among the group comprising perception, attention, learning, memory, thought, concept formation, reading, problem solving, and language.

12. The use according to claim 10, **characterised in that** said medicament is for the prevention and/or for the treatment of a person subject to, and/or intoxicated by agents with a cholinesterase inhibitory affinity.

13. The use according to claim 12, **characterised in that** said agents are selected from the group of organophosphates.

14. The use according to claim 10, **characterised in that** the pharmacologically active substance of general formula (I) comprised in said system is continuously release over a period of at least one month.

15. The use according to claim 14, **characterised in that** medicament is administrated to the patient at regular intervals lasting at least one month.

## Patentansprüche

1. Subkutanes Verabreichungssystem in der Form eines festen Implantats, welches ein Extrudat ist, umfassend eine biologisch abbaubare Polymermatrix und wenigstens einen der pharmazeutischen Wirkstoffe der allgemeinen Formel (I): 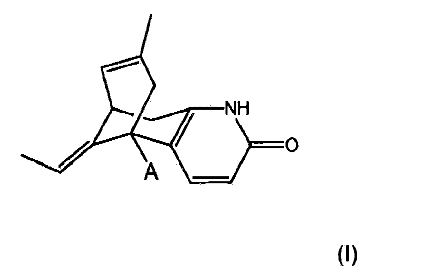 wobei der Rest A eine Aminogruppe -NH₂, eine Ammoniumgruppe -NH₃⁺ oder einen Rest der allgemeinen Formel (II) darstellt, 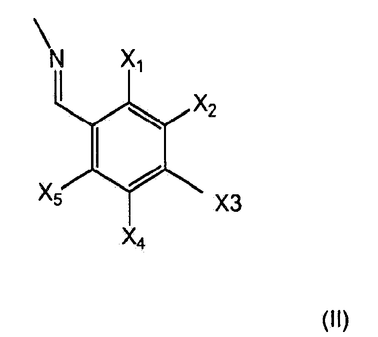 wobei jedes der X₁ bis X₅ unabhängig ein Wasserstoffatom, einen linearen oder verzweigten C₁- bis C₆-Alkylrest, einen linearen oder verzweigten C₁- bis C₆-Alkyloxyrest, eine Hydroxygruppe -OH, eine Aminogruppe -NH₂, einen primären oder sekundären C₁- bis C₆-Alkylaminorest, ein Halogenatom, eine Nitrogruppe - NO₂ darstellt; wobei der Stoff in der Matrix eingebettet ist.

2. Abgabesystem gemäß Anspruch 1, wobei in Formel (I), welche den pharmazeutischen Wirkstoff definiert, der Rest A eine Aminogruppe -NH₂, eine Ammoniumgruppe -NH₃⁺ oder einen Rest der allgemeinen Formel (II) darstellt, 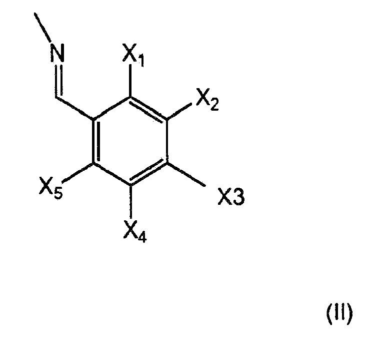 wobei jedes der X₁ bis X₅ unabhängig ein Wasserstoffatom, eine Methyl- oder Ethylgruppe, eine Methoxygruppe, eine Hydroxygruppe -OH, eine Aminogruppe -NH₂, eine Methylaminogruppe oder eine Dimethylaminogruppe, ein Chloratom, ein Bromatom oder eine Nitrogruppe -NO₂ darstellt.

3. Abgabesystem gemäß Anspruch 1, wobei der pharmazeutische Wirkstoff folgende Formel (III) aufweist:

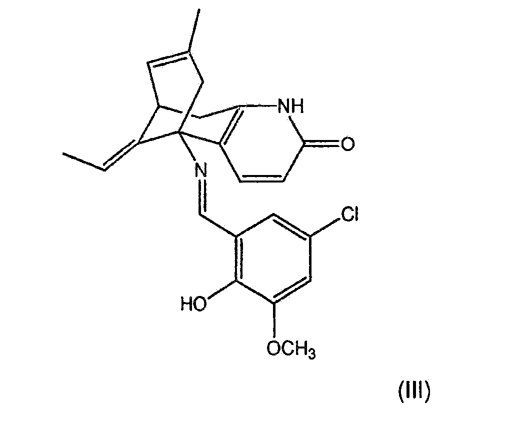

4. Abgabesystem gemäß Anspruch 1, wobei das biologisch abbaubare Polymermaterial der Matrix ausgewählt ist aus der Gruppe, umfassend Polyacetale und Poly(hydroxycarbonsäureester), Polyorthoester, Polyanhydride, Polylactone oder ein Gemisch davon.

5. Abgabesystem gemäß Anspruch 4, wobei die Poly(hydroxycarbonsäureester) ausgewählt sind aus der Gruppe, umfassend Homopolymere und Copolymere von D-Milchsäure und/oder L-Milchsäure und/oder Glycolsäure; und Blockpolymere davon mit Polyethylenglycol; und die Polylactone ausgewählt sind aus der Gruppe, umfassend Polycaprolacton, Poly(3-hydroxybutyrolacton) und Hydroxybutyrolacton/Hydroxyvalerolacton-Copolymer.

6. Abgabesystem gemäß Anspruch 4, wobei das biologisch abbaubare Polymermaterial ausgewählt ist aus der Gruppe, umfassend Polymilchsäure und Copolymere von D-Milchsäure und/oder L-Milchsäure und/oder D,L-Milchsäure und/oder Glycolsäure.

7. Abgabesystem gemäß Anspruch 6, wobei das biologisch abbaubare Polymermaterial ausgewählt ist aus der Gruppe, bestehend aus Poly(D,L-lactid-co-glycolid) und Polylactid, und das relative Verhältnis von D,L-Lactid zu Glycolid, die das biologisch abbaubare Polymermaterial bilden, im Bereich von 25:75 bis 100:0 Mol-%, vorzugsweise 50:50 bis 75:25 Mol-% liegt; wobei die Eigenviskosität des biologisch abbaubaren Polymermaterials im Bereich von 0,10 bis 0,9 dL/g, vorzugsweise 0,15 bis 0,6 dL/g, liegt.

8. Abgabesystem gemäß Anspruch 1, wobei das feste Implantat eine zylindrische Form aufweist.

9. Verfahren zur Herstellung des in Anspruch 4 definierten Abgabesystems, umfassend folgende Schritte:
a) Herstellen eines homogenen Gemischs aus einer geeigneten Menge eines trockenen Pulvers von wenigstens einem pharmazeutischen Wirkstoff der allgemeinen Formel (I) und einer geeigneten Menge eines trockenen Pulvers des biologisch abbaubaren Polymermaterials;
b) Extrudieren des in Schritt a) erhaltenen Gemischs mit einer kontrollierten Rate durch ein Formwerkzeug mit einem geeigneten kreisförmigen Querprofil bei Temperaturen im Bereich von 70 °C bis 100 °C;
c) Schneiden des in Schritt b) erhaltenen Extrudats im Wesentlichen durch das Querprofil des Extrudats zu einer geeigneten Länge; und
d) Sterilisieren der in Schritt c) erhaltenen Implantate.

10. Verwendung des in Anspruch 1 definierten Abgabesystems zur Herstellung eines Medikaments zur Behandlung einer Person, die an Störungen mit cholinergem Mangel leidet und/oder zur Verbesserung von Funktionen mit cholinerger Abhängigkeit bei einer Person.

11. Verwendung gemäß Anspruch 10, wobei die Störungen die Alzheimersche Erkrankung, schwache kognitive Beeinträchtigung, Myasthenia gravis, Demenz und Demenz mit vaskulärem Ursprung umfassen; und diese kognitiven Funktionen bei Vorgängen im Menschen beteiligt sind, die ausgewählt sind aus der Gruppe, umfassend Wahrnehmung, Aufmerksamkeit, Lernen, Gedächtnis, Denken, Konzeptbildung, Lesen, Problemlösung und Sprache.

12. Verwendung gemäß Anspruch 10, wobei das Medikament zur Vorbeugung und/oder zur Behandlung einer Person, die Mitteln mit einer cholinesterasehemmenden Wirkung ausgesetzt und/oder damit vergiftet ist, vorgesehen ist.

13. Verwendung gemäß Anspruch 12, wobei die Mittel aus der Gruppe der Organophosphate ausgewählt sind.

14. Verwendung gemäß Anspruch 10, wobei der pharmazeutische Wirkstoff der allgemeinen Formel (I), der in dem System enthalten ist, über einen Zeitraum von wenigstens einem Monat kontinuierlich freigesetzt wird.

15. Verwendung gemäß Anspruch 14, wobei das Medikament dem Patienten in regelmäßigen Intervallen mit Dauern von wenigstens einem Monat verabreicht wird.

## Revendications

1. Système de libération sous-cutané sous la forme d'un implant solide qui est un produit extrudé comprenant une matrice polymère biodégradable et au moins une des substances pharmacologiquement actives de formule générale (I) : 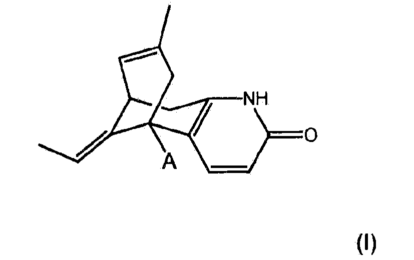 dans laquelle le résidu A représente un groupe amino -NH₂ ou un groupe ammonium -NH₃⁺ ou un résidu de formule générale (II) : 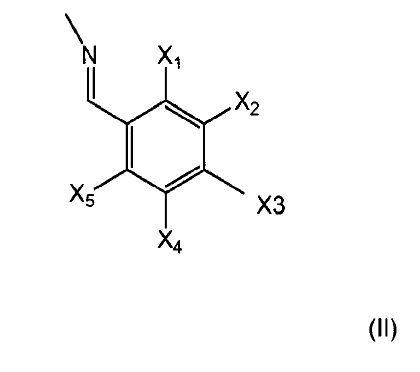 dans laquelle chaque X₁ à X₅ représente, indépendamment, un atome d'hydrogène, un groupe alkyle en C₁ à C₆ linéaire ou ramifié, un groupe alkyloxy en C₁ à C₆ linéaire ou ramifié, un groupe hydroxyle -OH, un groupe amino -NH₂, un groupe alkylamino en C₁ à C₆ primaire ou secondaire, un atome d'halogène, un groupe nitro -NO₂ ; ladite substance étant enrobée dans ladite matrice.

2. Système de libération selon la revendication 1,
**caractérisé en ce que** dans la formule générale (I) définissant ladite substance pharmacologiquement active, le résidu A représente un groupe amino -NH₂ ou un groupe ammonium -NH₃⁺ ou un résidu de formule générale (II) : 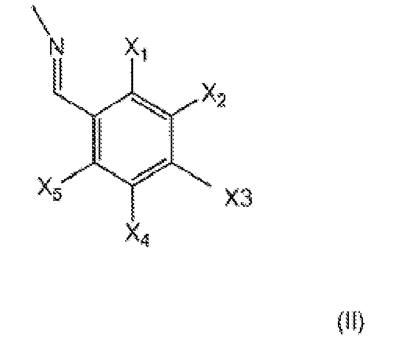 dans laquelle chaque X₁ à X₅ représente, indépendamment, un atome d'hydrogène, un groupe méthyle ou éthyle, un groupe méthoxy, un groupe hydroxyle -OH, un groupe amino -NH₂, un groupe méthylamino ou un groupe diméthylamino, un atome de chlore ou de brome, un groupe nitro -NO₂.

3. Système de libération selon la revendication 1, **caractérisé en ce que** ladite substance pharmacologiquement active présente la formule suivante (III) :

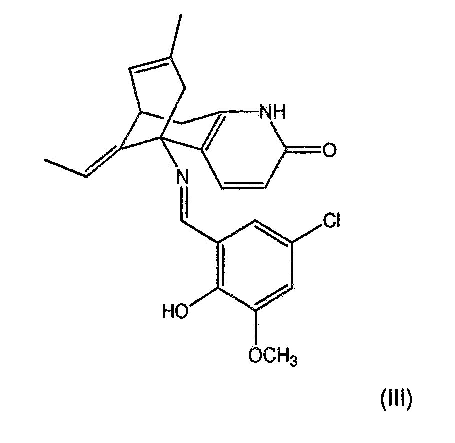

4. Système de libération selon la revendication 1, **caractérisé en ce que** le matériau polymère biodégradable de ladite matrice est choisi dans le groupe comprenant : les polyacétals et poly(esters hydroxycarboxyliques), les polyorthoesters, les polyanhydrides, les polylactones, ou un mélange de ceux-ci.

5. Système de libération selon la revendication 4, **caractérisé en ce que** lesdits poly(esters hydroxycarboxyliques) sont choisis dans le groupe comprenant les homopolymères et les copolymères de l'acide D-lactique et/ou L-lactique et/ou glycolique ; et les polymères à blocs de ceux-ci avec du polyéthylèneglycol ; et lesdites polylactones sont choisies dans le groupe comprenant la polycaprolactone, la poly(3-hydroxybutyrolactone), et un copolymère d'hydroxybutyrolactone-hydroxyvalérolactone.

6. Système de libération selon la revendication 4, **caractérisé en ce que** ledit matériau polymère biodégradable est choisi dans le groupe comprenant le poly(acide lactique) et les copolymères d'acide D-lactique et/ou L-lactique et/ou D,L-lactique et/ou glycolique.

7. Système de libération selon la revendication 6, **caractérisé en ce que** ledit matériau polymère biodégradable est choisi dans le groupe constitué par le poly(D,L-lactide-co-glycolide) et le polylactide et la proportion entre le D,L-lactide et le glycolide constituant le matériau polymère biodégradable est comprise dans la plage de 25:75 à 100:0% en mole, de préférence de 50:50 à 75:25% en mole ; la viscosité inhérente du matériau polymère biodégradable est comprise dans la plage de 0,10 à 0,9 dl/g, de préférence de 0,15 à 0,6 dl/g.

8. Système de libération selon la revendication 1, **caractérisé en ce que** ledit implant solide présente une forme cylindrique.

9. Procédé pour la préparation du système de libération tel que défini dans la revendication 4 comprenant les étapes suivantes :
a) préparation d'un mélange homogène d'une quantité appropriée de poudre sèche de ladite une des substances pharmacologiquement actives de formule générale (I) et d'une quantité appropriée de poudre sèche dudit matériau polymère biodégradable ;
b) extrusion du mélange tel qu'obtenu dans l'étape a) à une vitesse contrôlée via une filière avec une section circulaire appropriée placée à des températures allant de 70°C à 100°C ;
c) découpe du produit extrudé comme obtenu dans l'étape b) essentiellement via la section dudit produit extrudé et à une longueur appropriée ; et
d) stérilisation des implants tels qu'obtenus dans l'étape c).

10. Utilisation du système de libération tel que défini dans la revendication 1 pour la préparation d'un médicament pour le traitement d'une personne souffrant de troubles de déficience cholinergique et/ou pour l'amélioration de fonctions cholinergiques dépendantes chez une personne.

11. Utilisation selon la revendication 10, **caractérisée en ce que** lesdits troubles comprennent la maladie d'Alzheimer, la dégradation cognitive modérée, la myasthénie gravis, la démence, la démence d'origine vasculaire ; et lesdites fonctions de cognition sont impliquées dans des processus chez les êtres humains choisis dans le groupe comprenant la perception, l'attention, l'apprentissage, la mémoire, la pensée, la formation de concepts, la lecture, la résolution de problèmes et le langage.

12. Utilisation selon la revendication 10, **caractérisée en ce que** ledit médicament est également prévu pour la prévention et/ou pour le traitement d'une personne sujette à et/ou intoxiquée par des agents avec une activité inhibitrice de la cholinestérase.

13. Utilisation selon la revendication 12, **caractérisée en ce que** lesdits agents sont choisis dans le groupe des organophosphates.

14. Utilisation selon la revendication 10, **caractérisée en ce que** la substance pharmacologiquement active de formule générale (I) comprise dans ledit système est libérée en continu sur une période d'au moins un mois.

15. Utilisation selon la revendication 14, **caractérisée en ce que** le médicament est administré au patient à des intervalles réguliers durant au moins un mois.
